Europäisches Patentamt

European Patent Office

Office européen des brevets

(19)

(11) Numéro de publication: **0 278 247**
**A2**

(12) # DEMANDE DE BREVET EUROPEEN

(21) Numéro de dépôt: **88100423.8**

(22) Date de dépôt: **14.01.88**

(51) Int. Cl.4: **A61M 5/315**

(30) Priorité: **11.02.87 CH 511/87**

(43) Date de publication de la demande:
**17.08.88 Bulletin 88/33**

(84) Etats contractants désignés:
**AT BE DE FR GB IT NL SE**

(71) Demandeur: **CAMECO MEDICAL LIMITED**
**Chelsea Chambers 262A Fulham Road**
**London SW10 9EL(GB)**

(72) Inventeur: **Swallert, Sven Arild**
**11, rue Michel-Chauvet**
**CH-1208 Genève(CH)**

(74) Mandataire: **Micheli & Cie**
**118, rue du Rhône Case postale 47**
**CH-1211 Genève 6(CH)**

(54) **Dispositif de support et de manipulation d'une seringue.**

(57) Le dispositif de support et de manipulation d'une seringue comporte une poignée (1), une portion de support (2), deux tiges de guidage (3) parallèles reliant rigidement la poignée et la portion de support, et un organe de préhension (10) monté coulissant axialement entre les deux tiges de guidage et tournant sur lui-même autour de son axe central. Une plaque de fixation (7) présentant un orifice central est montée déplaçable parallèlement à la surface supérieure de la plaque de support (4) et est soumise à l'action de ressorts (8) tendant à maintenir cette plaque de fixation en contact avec ladite plaque de support de telle sorte qu'en position de service le rebord périphérique (13') du corps cylindrique (13) de la seringue soit maintenu par serrage entre la plaque de fixation et la plaque de support. Enfin, l'organe de préhension présente des ouvertures d'accouplement (11,11') coopérant en position de service avec l'extrémité supérieure (14') du piston (14) de la seringue.

FIG. 1

## Dispositif de support et de manipulation d'une seringue

La présente invention concerne un dispositif de support et de manipulation d'une seringue.

Il est connu d'utiliser des seringues du type classique pour pratiquer des biopsies par aspiration, en vue d'un examen diagnostique d'une substance déterminée prélevée dans le corps d'un être vivant. Cette technique est notamment utilisée pour des ponctions lombaires, prostatiques, amniotiques, pour l'examen de tumeurs, etc. Selon la nature et la consistance de la substance à prélever, le mouvement de retrait du piston de la seringue nécessite plus ou moins de force. De plus, l'extrémité libre du piston de la seringue n'offre pas de moyens adéquats pour exercer cette force de manière aussi axiale que possible. C'est pourquoi, il s'avère utile, voire nécessaire, de disposer de moyens permettant à l'utilisateur d'actionner de manière plus précise le piston de la seringue.

Le but de cette invention consiste donc à fournir un dispositif de support et de manipulation d'une seringue qui offre les possibilités ci-dessus, et qui puisse être utilisé avec des seringues de dimensions différentes.

Le dispositif de support et de manipulation d'une seringue selon l'invention, qui vise à atteindre le but précité, présente les caractéristiques énoncées dans la revendication 1.

Le dessin annexé illustre schématiquement et à titre d'exemple une forme d'exécution du dispositif selon l'invention.

La figure 1 en est une vue de face partiellement en coupe, avec une seringue en position de service.

La figure 2 en est une vue de côté.

La figure 3 en est une vue de face, également avec une seringue en position de service.

En référence au dessin annexé, le dispositif selon l'invention illustré comporte une poignée 1, une portion de support 2 et deux tiges de guidage 3 parallèles reliant rigidement la poignée et la portion de support. Cette dernière comporte une plaque de base 4 percée d'un orifice central et prolongé vers le bas par une jupe de positionnement 5 partiellement cylindrique.

Les parties inférieures 6 des tiges de guidage 3 présentent un diamètre inférieur au reste des tiges et des filetages 6' leur extrémité, vissées dans des taraudages pratiqués dans la plaque de base 4 de la portion de support 2.

En outre, une plaque de fixation 7 est montée coulissante axialement entre les deux tiges de guidage 3, plus particulièrement sur les parties inférieures 6 de celles-ci, et est soumise à l'action de ressorts 8 montés sur ces parties inférieures 6 et tendant à maintenir la plaque de fixation 7 contre la face supérieure de la plaque de base 4 de la portion de support 2. Cette plaque de base 4 peut être de plus munie sur cette face supérieure d'une plaquette d'appui 9, de préférence réalisée en un matériau semi-rigide.

Enfin, le dispositif comporte un organe de préhension 10 monté entre les deux tiges de guidage 3 de manière à être librement déplaçable axialement par coulissement sur lesdites tiges (flèche I) et pivotant librement sur lui-même autour de son axe central entre celles-ci (flèche II). Cet organe de préhension présente une forme générale circulaire, avec deux ouvertures radiales 11,11' de dimensions différentes et diamétralement opposées présentant des encoches transversales. Entre ces ouvertures radiales 11,11', l'organe de préhension 10 comporte sur sa tranche périphérique deux rainures de guidage 12 destinées à coopérer avec les tiges de guidage 3 pour permettre ces mouvements respectivement de translation axiale (I) et de rotation (II) comme décrit ci-dessus.

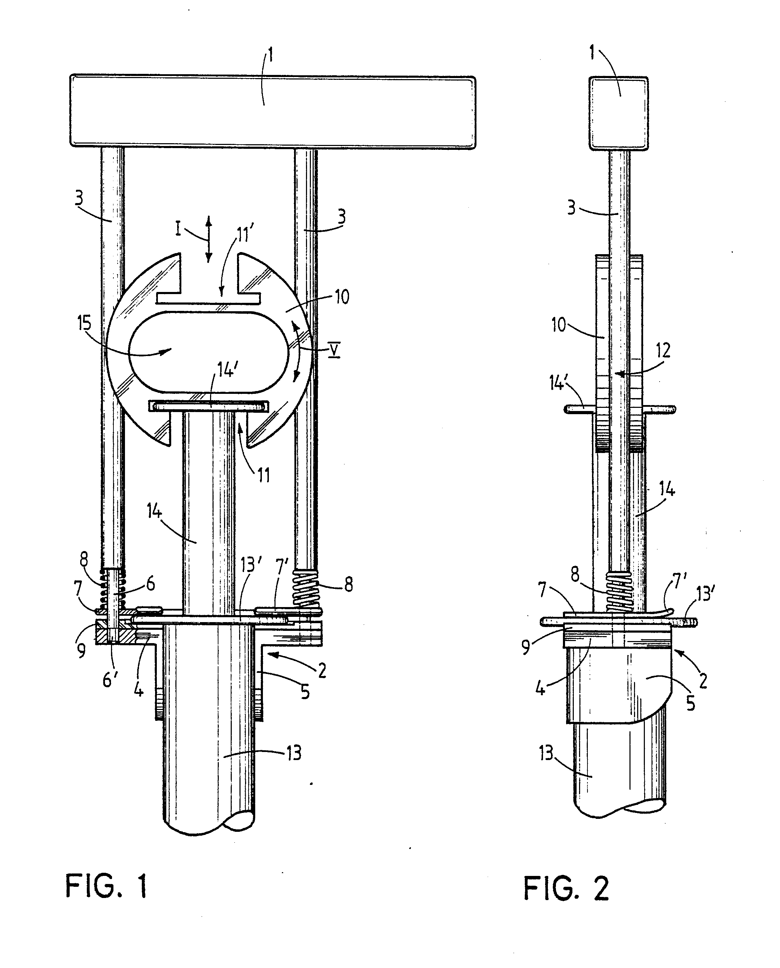

Comme illustré sur la figure 1, une seringue 13 peut être fixée en position de service, par coincement du rebord périphérique 13' situé à l'extrémité supérieure du corps cylindrique de la seringue entre la plaque de fixation 7 et la plaquette d'appui 9, grâce à l'action du ressort 8. Pour faciliter l'introduction dudit rebord 14 entre la plaque de fixation 7 et la plaquette d'appui 9, le bord antérieur 7' de la plaque de fixation 7 est légèrement relevé (voir figure 2). Quand à la partie supérieure du corps cylindrique de la seringue 13, elle est disposée dans cette position de service dans la portion cylindrique de la jupe de positionnement 5. Dans la position de service illustrée, l'extrémité libre du piston 14 de la seringue 13 est maintenue par un rebord périphérique 14' dans l'encoche 11 de l'organe de préhension prévue à cet effet.

Pour l'utilisateur il convient alors, avec une seule main, de tenir la poignée 1 et simultanément avec deux doigts, par exemple, introduits dans l'ouverture centrale 15 que présente l'organe de préhension 10, de tirer celui-ci en direction de ladite poignée 1, de manière à provoquer par le retrait du piston 14 une aspiration dans le corps cylindrique de la seringue 13. Généralement l'extrémité inférieure de la seringue 13 est munie directement d'une aiguille creuse (non montrée) ou bien reliée par un tuyau à une telle aiguille creuse, celle-ci étant introduite dans une zone particulière du corps vivant de laquelle l'utilisateur souhaite prélever une substance destinée à être analysée en vue de l'établissement d'un diagnostic par exemple.

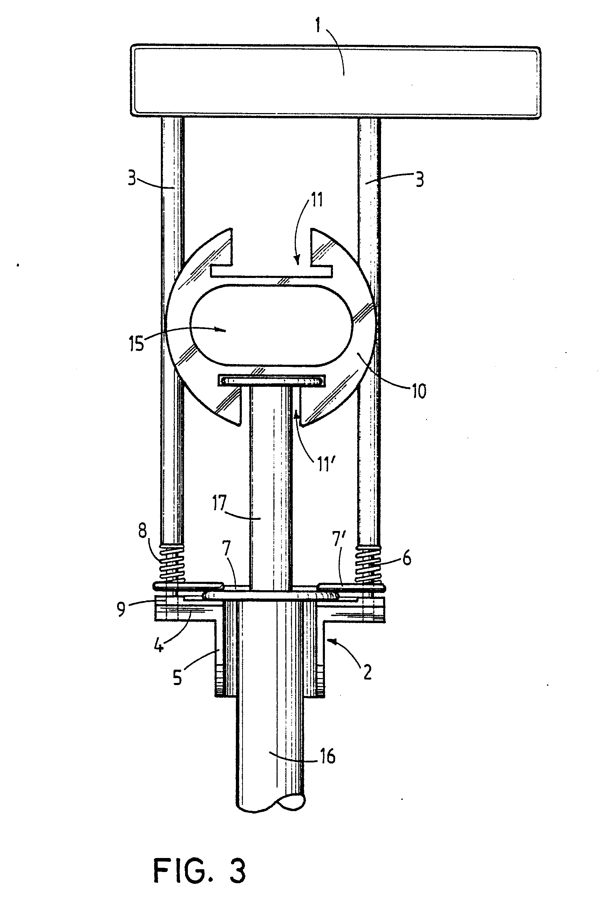

Comme illustré sur la figure 3, la conception du dispositif selon l'invention permet l'utilisation de seringues de dimensions différentes. Grâce à la présence de la plaque de fixation 7, une seringue 16 de dimensions plus petites que celles de la seringue 13 de la figure 1 peut être fixée sans problème en position de service dans la jupe de positionnement 5, dont le diamètre de la portion cylindrique est nettement supérieur à celui du corps cylindrique de cette seringue 16. De plus, dans cette réalisation, le rebord périphérique de l'extrémité du piston 17 est disposé dans l'encoche 11' de l'organe de préhension 10 qui est de dimension inférieure.

Les principaux avantages du dispositif selon la présente invention consistent dans le fait qu'il permet une manipulation précise du piston de la seringue d'une part, et qu'il est utilisable avec des types de seringues présentant des dimensions différentes.

## Revendications

1. Dispositif de support et de manipulation d'une seringue, caractérisé par le fait qu'il comporte une poignée, une portion de support, deux tiges de guidage parallèles reliant rigidement la poignée et la portion de support, et un organe de préhension monté coulissant axialement entre les deux tiges de guidage et tournant sur lui-même autour de son axe central, par le fait que la portion de support comporte une plaque de support percée d'un orifice central prolongé vers le bas par une jupe de positionnnement partiellement cylindrique et destinée à revenir en position de service sur la partie supérieure du corps cylindrique de la seringue, par le fait qu'une plaque de fixation présentant également un orifice central est montée déplaçable parallèlement à la surface supérieure de la plaque de support et soumise à une action élastique tendant à maintenir cette plaque de fixation en contact avec ladite plaque de support de telle sorte qu'en position de service le rebord périphérique du corps cylindrique de la seringue soit maintenu par serrage entre la plaque de fixation et la plaque de support, et par le fait que l'organe de préhension présente des moyens d'accouplement d'au moins deux dimensions différentes et destinées à coopérer en position de service avec l'extrémité supérieure du piston de la seringue.

2. Dispositif selon la revendication 1, caractérisé par le fait que l'organe de préhension est de forme générale circulaire et que les moyens d'accouplement sont constitués par au moins deux ouvertures radiales de dimensions différentes et présentant des encoches destinées à coopérer en position de service avec le rebord périphérique de l'extrémité libre du piston de la seringue.

3. Dispositif selon la revendication 2, caractérisé par le fait que l'organe de préhension présente sur sa tranche et entre les ouvertures radiales deux rainures de guidage en prise avec les tiges de guidage parallèles de manière à coulisser librement sur celles-ci et à tourner librement sur elles-mémes entre ces tiges.

4. Dispositif selon la revendication 2 ou la revendication 3, caractérisé par le fait que l'organe de préhension est percé d'une ouverture centrale destinée en position de service à permettre l'introduction d'au moins deux doigts de l'utilisateur en vue de la manipulation du piston de la seringue.

5. Dispositif selon l'une des revendications 1 à 4, caractérisé par le fait que la plaque de fixation est montée coulissante sur les deux tiges de guidage et qu'elle est soumise à l'action de ressorts disposés autour de celles-ci.

6. Dispositif selon la revendication 5, caractérisé par le fait que la plaque de fixation présente un bord antérieur relevé.

7. Dispositif selon l'une des revendications 1 à 6, caractérisé par le fait qu'une plaquette d'appui en un matériau semi-rigide est disposée sur la surface supérieure de la plaque de support.

FIG. 1          FIG. 2

0 278 247

FIG. 3